# EUROPEAN PATENT APPLICATION

(11) **EP 4 770 082 A1**
(43) Date of publication of application: **01.07.2026**
(21) Application number: 24891199.2
(22) Date of filing: 28.10.2024
(51) Int. Cl.: H04N 13/293, A61B 6/00, A61B 6/03, A61B 6/46, H04N 13/275, H04N 13/302, H04N 13/366

(54) **INFORMATION PROCESSING DEVICE, INFORMATION PROCESSING METHOD, PROGRAM, AND INFORMATION PROCESSING SYSTEM**

(30) Priority: 17.11.2023 JP 2023195911
(71) Applicant: Sony Group Corporation, Tokyo 108-0075 (JP)
(72) Inventor: KATSUKI Shinji, Tokyo 108-0075 (JP); NAKAYAMA Koharu, Tokyo 108-0075 (JP); BABA Hiroyasu, Tokyo 108-0075 (JP); FUKAZAWA Takanori, Tokyo 108-0075 (JP); NAKAMURA Takuya, Tokyo 108-0075 (JP)
(74) Representative: D Young & Co LLP
(86) International application number: PCT/JP2024/038298
(87) International publication number: WO 2025/105158

(57) **Abstract**

The present technology relates to an information processing apparatus, an information processing method, a program, and an information processing system that can suitably provide images to both surgeons and persons other than surgeons.
The information processing apparatus of the present technology includes an image generation unit that generates a first display image based on a viewing position of a user of a spatial reproduction display, the first display image being an image in which a 2D image is displayed in 2D on a display surface and a 3D model is displayed in 3D, and being an image displayed on the spatial reproduction display having the display surface. The present technology can be applied, for example, to surgical systems.

## Description

### [Technical Field]

The present technology relates to an information processing apparatus, an information processing method, a program, and an information processing system, and particularly relates to an information processing apparatus, an information processing method, a program, and an information processing system that can suitably provide images to both surgeons and persons other than surgeons.

### [Background Art]

IVR (Interventional Radiology) treatment, which involves performing vascular catheter treatment while viewing real-time angiographic fluoroscopic images captured using techniques such as DSA (Digital Subtraction Angiography), is commonly performed.

In IVR treatment, by displaying CG images generated based on CT (Computed Tomography) images or MRI (Magnetic Resonance Imaging) images captured in advance alongside angiographic fluoroscopic images or superimposed on angiographic projection images, surgeons can know the direction and angle of vascular pathways ahead of the current catheter position, and it is expected that treatment can be performed more safely and efficiently.

The displayed CG images are images rendered as 2D images from 3D models showing three-dimensional shapes of blood vessels and the like. If 3D models of blood vessels and angiographic fluoroscopic images are displayed on a spatial reproduction display capable of performing 3D display where 3D models are visually recognized as three-dimensional objects, as described in Patent Document 1, surgeons can recognize the shape of blood vessels more three-dimensionally.

### [Citation List]

### [Patent Documents]

[Patent Document 1]International Publication No. WO2023/140120

### [Summary]

### [Technical Problem]

When displaying 3D models of blood vessels and angiographic fluoroscopic images on a spatial reproduction display, the spatial reproduction display typically tracks only the surgeon's viewing position to perform 3D display, so for assistants and nurses around the surgeon, angiographic fluoroscopic images appear blurred.

The present technology has been made in view of such circumstances, and enables suitable provision of images to both surgeons and persons other than surgeons.

### [Means for Solving the Problem]

An information processing apparatus according to a first aspect of the present technology includes an image generation unit that generates a first display image based on a viewing position of a user of a spatial reproduction display, the first display image being an image in which a 2D image is displayed in 2D on a display surface and a 3D model is displayed in 3D, and being an image displayed on the spatial reproduction display having the display surface.

An information processing method according to the first aspect of the present technology is an information processing method for generating a first display image based on a viewing position of a user of a spatial reproduction display, the first display image being an image in which a 2D image is displayed in 2D on a display surface and a 3D model is displayed in 3D, and being an image displayed on the spatial reproduction display having the display surface.

A program according to the first aspect of the present technology is a program for causing a computer to execute processing for generating a first display image based on a viewing position of a user of a spatial reproduction display, the first display image being an image in which a 2D image is displayed in 2D on a display surface and a 3D model is displayed in 3D, and being an image displayed on the spatial reproduction display having the display surface.

An information processing system according to a second aspect of the present technology includes an information processing apparatus having an image generation unit that generates a first display image based on a viewing position of a user, the first display image being an image in which a 2D image is displayed in 2D on a display surface and a 3D model is displayed in 3D, and a spatial reproduction display having the display surface and displaying the first display image.

In the first aspect of the present technology, a first display image is generated based on a viewing position of a user of a spatial reproduction display, the first display image being an image in which a 2D image is displayed in 2D on a display surface and a 3D model is displayed in 3D, and being an image displayed on the spatial reproduction display having the display surface.

In the second aspect of the present technology, a first display image is generated by an information processing apparatus based on a viewing position of a user, the first display image being an image in which a 2D image is displayed in 2D on a display surface and a 3D model is displayed in 3D, and the first display image is displayed by a spatial reproduction display having the display surface.

### [Brief Description of Drawings]

[FIG. 1] A diagram showing a configuration example of a surgical system according to an embodiment of the present technology.
[FIG. 2] A diagram showing an example of a display surface of a spatial reproduction display.
[FIG. 3] A first diagram showing a display example of a surgical field image.
[FIG. 4] A second diagram showing a display example of a surgical field image.
[FIG. 5] A diagram showing an example of a display method for angiographic fluoroscopic images.
[FIG. 6] A diagram showing another example of a display method for angiographic fluoroscopic images.
[FIG. 7] A diagram showing an arrangement example of 3D models and angiographic fluoroscopic images.
[FIG. 8] A diagram showing a display example of 3D models and angiographic fluoroscopic images.
[FIG. 9] A flowchart explaining processing performed by an information processing apparatus.
[FIG. 10] A diagram showing an example of resolution of images displayed on a spatial reproduction display.
[FIG. 11] A flowchart explaining processing performed by an information processing apparatus when displaying high-resolution 2D images.
[FIG. 12] A diagram explaining 2D images that can be viewed by surgeons and persons other than surgeons in each display method.
[FIG. 13] A diagram showing a configuration example of a C-arm.
[FIG. 14] A diagram showing an arrangement example of 3D objects in virtual space.
[FIG. 15] A diagram showing an example of changing the orientation of 3D objects.
[FIG. 16] A diagram showing another example of arrangement of 3D objects in virtual space.
[FIG. 17] A diagram showing an actual display example of 3D objects.
[FIG. 18] A flowchart explaining processing performed by an information processing apparatus when arranging 3D objects in a positional relationship corresponding to the positional relationship between a C-arm and a patient.
[FIG. 19] A diagram showing an example of a screen displayed on a display device.
[FIG. 20] A first diagram explaining the flow of transition from a parallel display screen to a superimposed display screen.
[FIG. 21] A second diagram explaining the flow of transition from a parallel display screen to a superimposed display screen.
[FIG. 22] A block diagram showing a configuration example of computer hardware.

### [Mode for Carrying Out the Invention]

Hereinafter, modes for carrying out the present technology will be described. The description will be given in the following order.
1. Overview of surgical system
2. First embodiment
3. Second embodiment
4. Third embodiment

### <1. Overview of Surgical System>

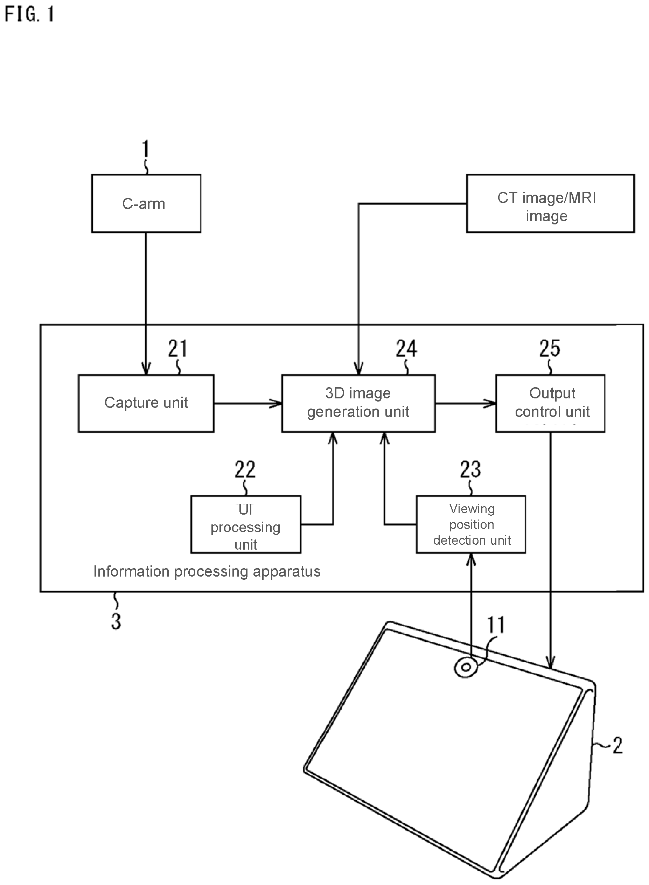

FIG. 1 is a diagram showing a configuration example of a surgical system according to an embodiment of the present technology.

The surgical system of FIG. 1 comprises a C-arm 1, a display device 2, and an information processing apparatus 3.

The C-arm 1 is an X-ray imaging apparatus that captures angiographic fluoroscopic images (X-ray fluoroscopic images) in which only the patient's blood vessels (at least a part of the living body) are depicted using X-ray techniques such as DSA.

The display device 2 is a display device that enables users to visually recognize virtual 3D objects arranged in a predetermined space as three-dimensional objects by displaying 3D images comprise left-eye images and right-eye images having parallax with each other, and is, for example, a spatial reproduction display.

The display device 2 is installed, for example, such that the display surface is perpendicular to the horizontal plane in real space, or such that the display surface faces diagonally upward with respect to the horizontal plane in real space. The display device 2 is provided with, for example, an imaging device 11 as a sensor used for detecting the user's viewing position.

The information processing apparatus 3 is a device that controls the entire surgical system of the present technology, and is, for example, a computer having an information processing circuit equipped with a CPU and memory. For example, the information processing apparatus 3 controls the display by the display device 2, thereby presenting angiographic fluoroscopic images to the user (surgeon).

The information processing apparatus 3 comprises a capture unit 21, a UI processing unit 22, a viewing position detection unit 23, a 3D image generation unit 24, and an output control unit 25.

The capture unit 21 captures captured images taken by the C-arm 1 to acquire angiographic fluoroscopic images, and supplies them to the 3D image generation unit 24. The angiographic fluoroscopic images are 2D images. Note that the capture unit 21 can also acquire surgical field images in which at least a part of the patient's living body is captured as a subject by imaging devices such as general cameras, stereo cameras, surgical microscopes, and endoscopes. The angiographic fluoroscopic images and surgical field images can be said to be live-action images that are images actually captured of subjects by imaging devices.

The UI processing unit 22 acquires operation content input by the user using input devices (not shown) or gestures, and supplies it to the 3D image generation unit 24.

The viewing position detection unit 23 detects the user's viewing position (position and orientation of the viewpoint) based on captured images taken by the imaging device 11, and supplies the detection result to the 3D image generation unit 24.

The 3D image generation unit 24 acquires DICOM (Digital Imaging and Communications in Medicine) data such as CT images and MRI images captured before surgery or treatment, and generates 3D models showing three-dimensional shapes of at least a part of the patient's living body (for example, blood vessels) appearing in angiographic fluoroscopic images based on the DICOM data.

The 3D image generation unit 24 arranges the angiographic fluoroscopic images supplied from the capture unit 21 and the generated 3D models in virtual space. The 3D image generation unit 24 renders the virtual space based on the user's viewing position detected by the viewing position detection unit 23 to generate 3D images. For example, the 3D image generation unit 24 generates 3D images so that angiographic fluoroscopic images and 3D models can be appropriately viewed from the user's viewing position. Here, the arrangement of angiographic fluoroscopic images and 3D models is controlled according to the operation content supplied from the UI processing unit 22.

Note that slice data acquired as DICOM data may be arranged in virtual space as 3D models.

The 3D image generation unit 24 supplies the generated 3D images to the output control unit 25.

The output control unit 25 controls the display by the display device 2. Specifically, the output control unit 25 supplies the 3D images supplied from the 3D image generation unit 24 to the display device 2, and displays the left-eye images and right-eye images that comprises the 3D images. By delivering the left-eye images to the user's left eye and the right-eye images to the user's right eye, for example, angiographic fluoroscopic images and 3D models of blood vessels arranged side by side are presented to the user in real time.

The user inserts a catheter while viewing angiographic fluoroscopic images and performs endovascular treatments such as aneurysm embolization, stent graft insertion, and angioplasty. By viewing the 3D models of blood vessels, the user can proceed with treatment while understanding three-dimensionally how the vascular pathways ahead of the current catheter position are configured.

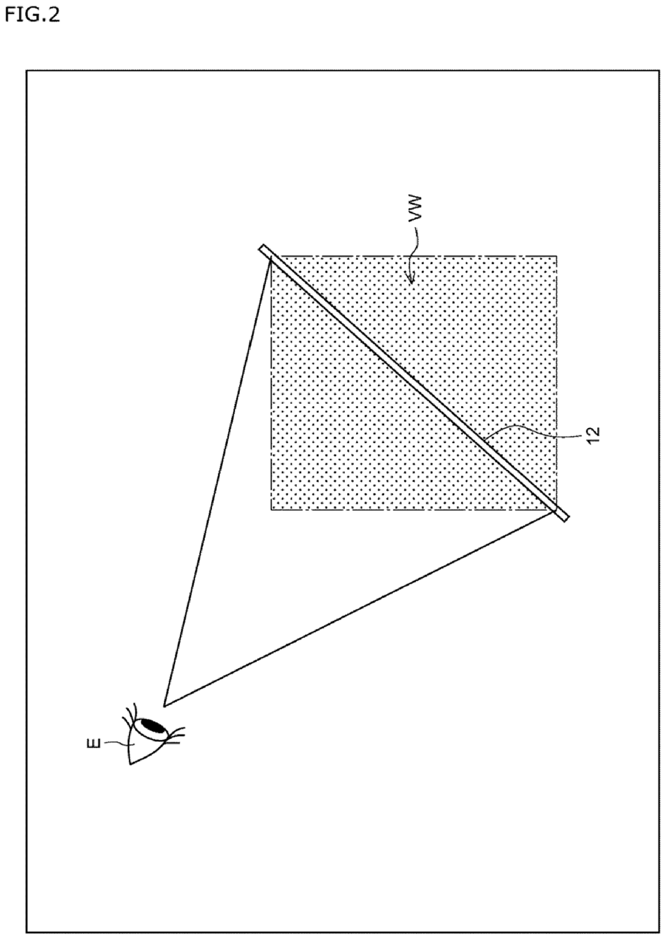

Next, the spatial reproduction display applied as the display device 2 of the surgical system will be described using FIGS. 2 to 4.

In recent years, naked-eye stereoscopic image display devices capable of displaying content three-dimensionally without using dedicated glasses have been proposed as spatial reproduction displays. Such spatial reproduction displays are capable of displaying images shifted horizontally for each viewing angle, and users can perceive depth through the displacement (parallax) between left-eye images and right-eye images.

Note that a spatial reproduction display using dedicated glasses may be applied as the display device 2.

FIG. 2 is a diagram showing an example of a display surface of a spatial reproduction display.

As described above, the spatial reproduction display is arranged, for example, such that the display surface 12 faces diagonally upward with respect to the horizontal plane in real space. In this case, in the spatial reproduction display, a virtual space VW is reproduced, for example, so as to intersect with the display surface 12, in other words, to have regions on both the front side and the back side of the display surface 12 as viewed from the user's viewing position E.

The 3D objects arranged in the virtual space VW are displayed, for example, so that a part appears to protrude from the display surface 12. The spatial reproduction display expresses virtual depth by displaying 3D images in which different images are delivered to the user's left eye and right eye, respectively, and can give the user a sensation as if 3D objects exist in real space. By arranging the display surface 12 to face diagonally upward with respect to the horizontal plane in real space, the spatial reproduction display can reproduce the virtual space VW in an easily understandable manner.

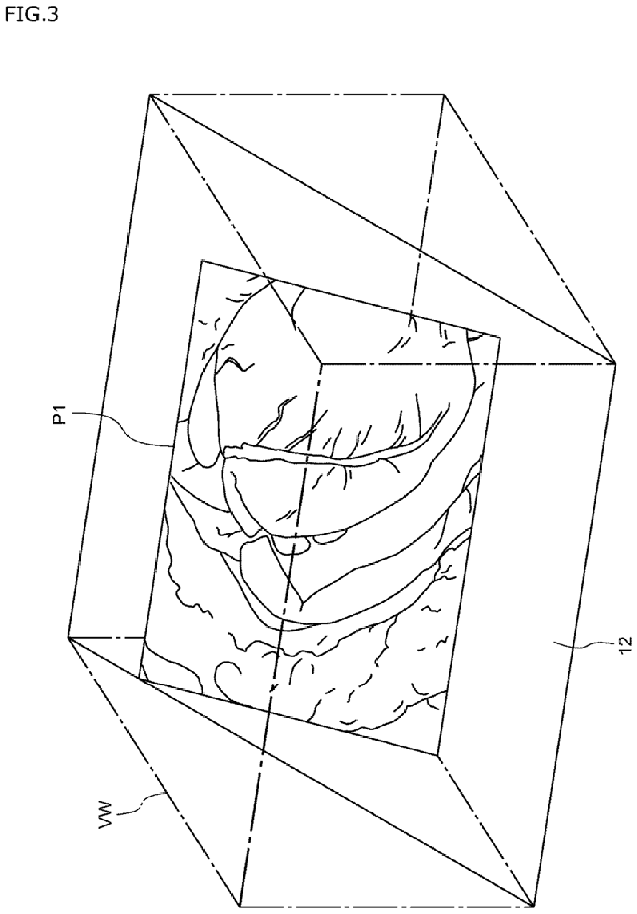

FIG. 3 is a diagram showing a display example of a surgical field image.

As shown in FIG. 3, the surgical field image P1 is displayed, for example, by being projected onto a virtual plate arranged in the virtual space VW. The virtual plate onto which the surgical field image P1 is projected is arranged so as to always face directly toward the user's viewing position.

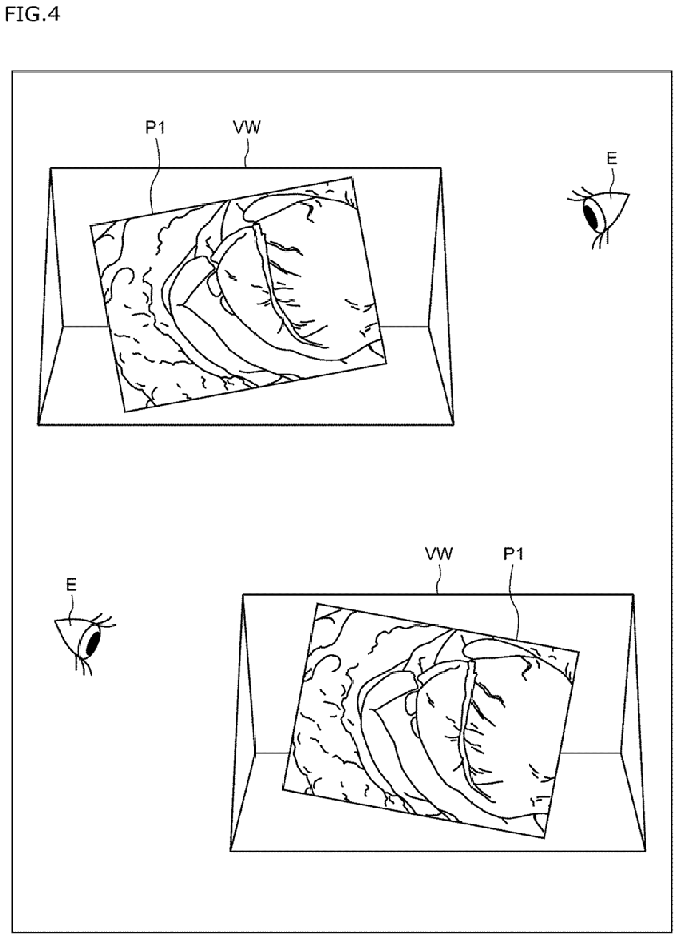

Therefore, as shown in the upper part of FIG. 4, when the user views the virtual space VW from the right side facing the spatial reproduction display (virtual space VW), the virtual plate onto which the surgical field image P1 is projected is arranged to face the right side. Also, as shown in the lower part of FIG. 4, when the user views the virtual space VW from the left side facing the spatial reproduction display, the virtual plate onto which the surgical field image P1 is projected is arranged to face the left side.

Angiographic fluoroscopic images are also displayed by being projected onto virtual plates in the same manner as surgical field images.

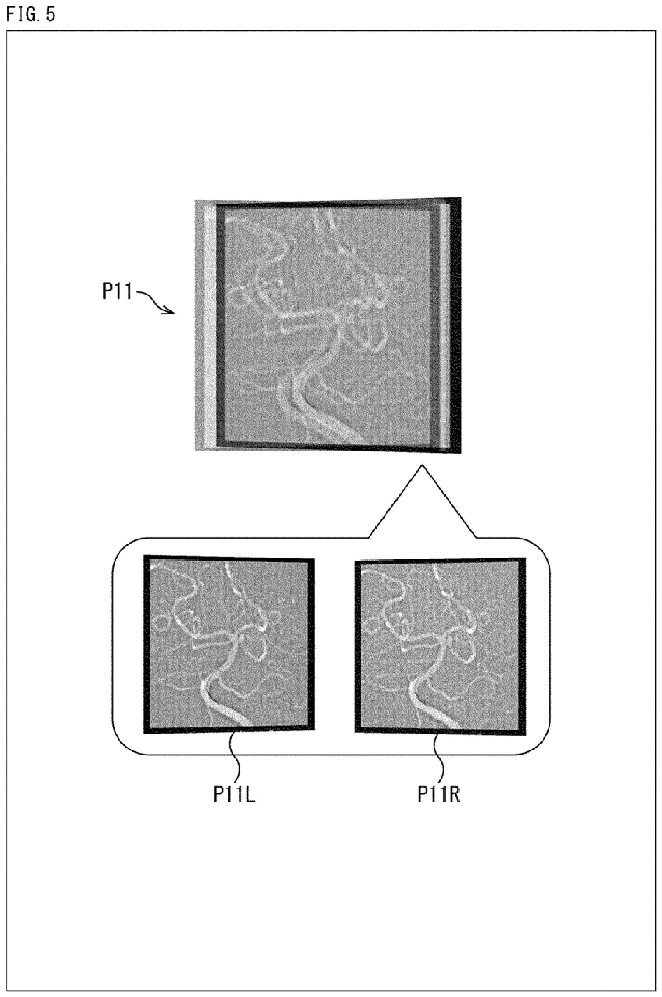

FIG. 5 is a diagram showing an example of a display method for angiographic fluoroscopic images.

As shown in the callout in FIG. 5, by displaying left-eye angiographic fluoroscopic image P11L and right-eye angiographic fluoroscopic image P11R having parallax with each other on the spatial reproduction display, the angiographic fluoroscopic image P11 appears to the user as being projected onto a virtual plate arranged in virtual space. The spatial reproduction display typically tracks the viewing position of only one person (for example, the surgeon) to perform 3D image display. Therefore, for assistants and nurses around the surgeon, the angiographic fluoroscopic image P11 appears blurred, as shown in the upper part of FIG. 5. Hereinafter, the display of left-eye images and right-eye images having parallax with each other is also referred to as 3D display.

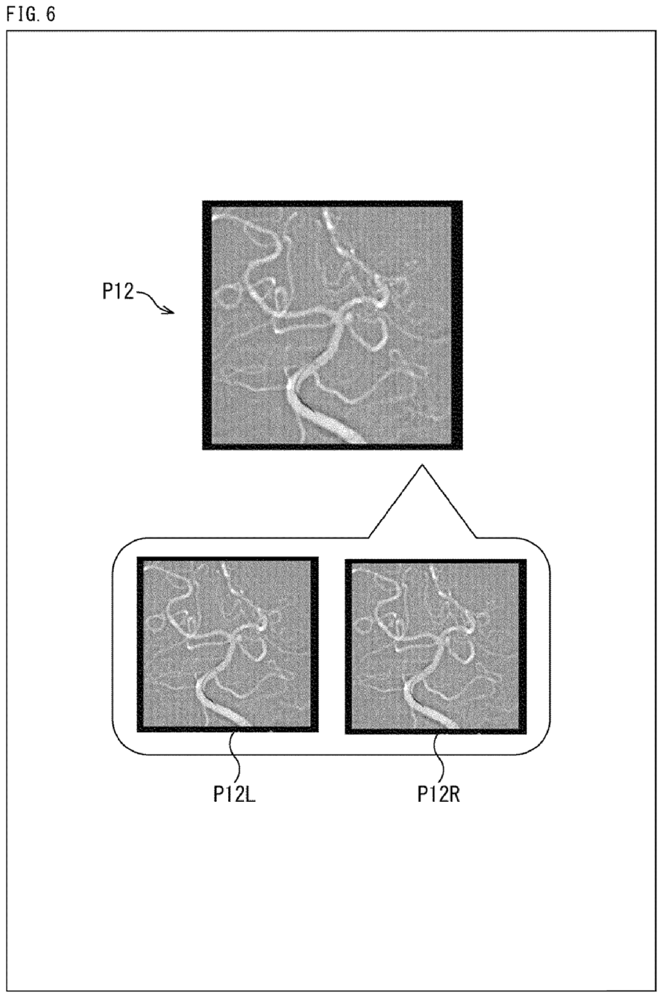

FIG. 6 is a diagram showing another example of a display method for angiographic fluoroscopic images.

As shown in the callout in FIG. 6, by displaying the same image as left-eye angiographic fluoroscopic image P12L and right-eye angiographic fluoroscopic image P12R on the spatial reproduction display, surgeons, assistants, nurses, and others can clearly see the angiographic fluoroscopic image P12, as shown in the upper part of FIG. 6. In this case, for surgeons, assistants, nurses, and others, the angiographic fluoroscopic image P12 appears to be displayed on the display surface 12 of the spatial reproduction display. Hereinafter, the display of the same image as left-eye images and right-eye images is also referred to as 2D display or display surface display.

### <2. First Embodiment>

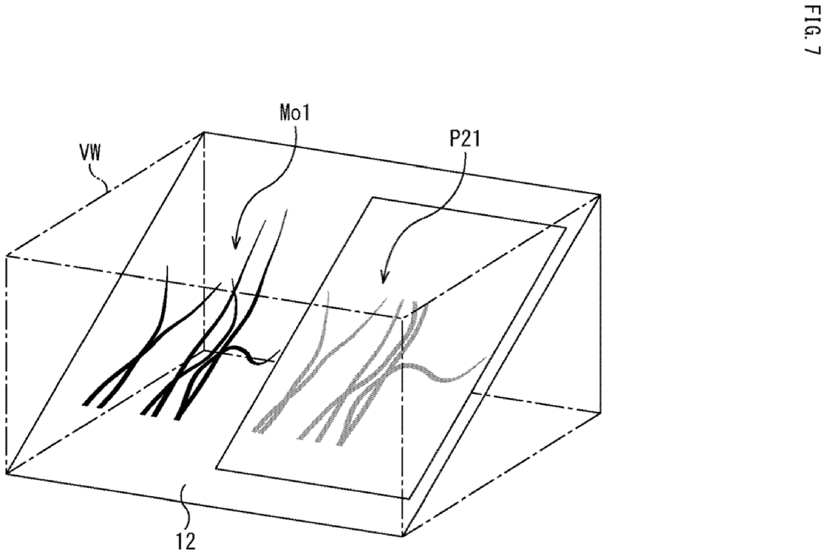

FIG. 7 is a diagram showing an arrangement example of 3D models and angiographic fluoroscopic images.

As shown in FIG. 7, when the 3D model Mo1 of blood vessels and the angiographic fluoroscopic image P21 are displayed side by side, in the virtual space VW, for example, the angiographic fluoroscopic image P21 projected onto a virtual plate is arranged next to the 3D model Mo1 of blood vessels as viewed from the viewing position of the angiographic fluoroscopic image P21 (the position of the X-ray generator of the C-arm 1).

In the present technology, the virtual plate onto which the angiographic fluoroscopic image P21 is projected is arranged on the display surface 12 of the display device 2. In other words, the angiographic fluoroscopic image P21 is displayed on the display surface 12. To put it another way, the regions where the angiographic fluoroscopic image P21 appears in the left-eye images and right-eye images displayed on the display device 2 become the same image.

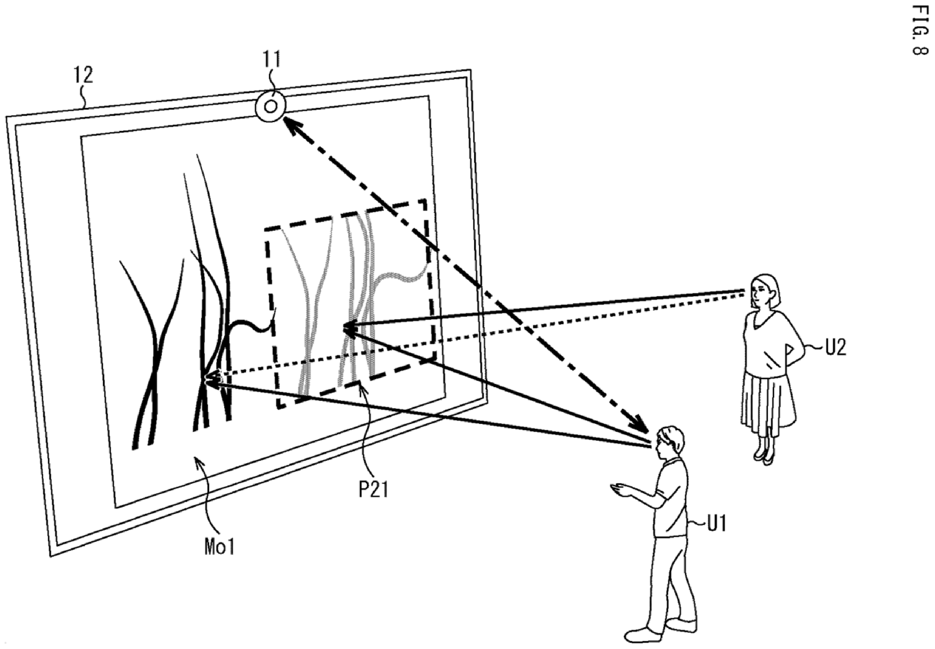

FIG. 8 is a diagram showing a display example of 3D models and angiographic fluoroscopic images.

In the display device 2, as shown by the dashed-dotted arrow in FIG. 8, the 3D model Mo1 arranged in virtual space is displayed in 3D based on the viewing position of the surgeon U1 tracked using the imaging device 11. Therefore, as shown by connecting the surgeon U1 and the 3D model Mo1 with a solid arrow, the surgeon U1 can clearly and appropriately visually recognize the 3D model Mo1 as a three-dimensional object. On the other hand, as shown by connecting the assistant U2 and the 3D model Mo1 with a dotted arrow, for the assistant U2, the 3D model Mo1 appears blurred or distorted.

Also, in the display device 2, the angiographic fluoroscopic image P21 is displayed on the display surface 12. Therefore, as shown by connecting the surgeon U1 and the angiographic fluoroscopic image P21 with a solid arrow, the surgeon U1 can clearly visually recognize the angiographic fluoroscopic image P21. On the other hand, as shown by connecting the assistant U2 and the angiographic fluoroscopic image P21 with a solid arrow, the assistant U2 can also visually recognize the angiographic fluoroscopic image P21.

In the display device 2, as shown by surrounding the angiographic fluoroscopic image P21 with a dashed line in FIG. 8, the region (image) displayed on the display surface 12 is highlighted, thereby clearly indicating that 2D images can be clearly seen even from viewing positions that are not being tracked. This allows persons other than the surgeon to easily discover images that appear clear to them and to focus on clearly visible images while ignoring the 3D model Mo1 that appears blurred.

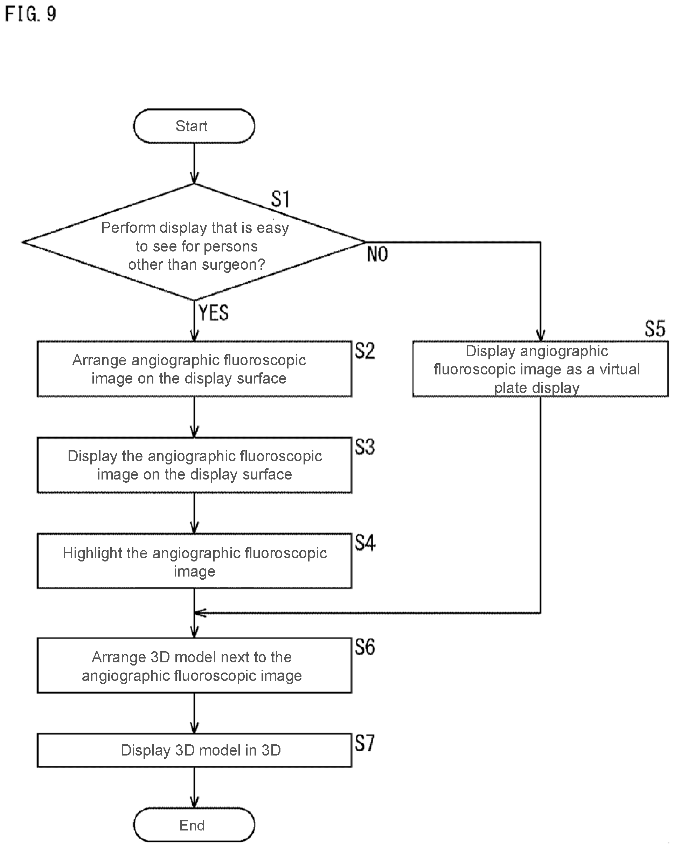

Next, with reference to the flowchart of FIG. 9, the processing performed by the information processing apparatus 3 having the above configuration will be described. Here, the acquisition of angiographic fluoroscopic images and 3D models of blood vessels, the acquisition of operation content, and the detection of the user's viewing position are performed as appropriate.

In step S1, the information processing apparatus 3 determines whether to perform display that is easy to see for persons other than the surgeon. Whether to perform display that is easy to see for persons other than the surgeon is determined, for example, based on operation content by the surgeon. It is also possible to determine whether there are people around the surgeon based on captured images taken by the imaging device 11, and when persons other than the surgeon are around the surgeon, to determine that display that is easy to see for persons other than the surgeon may be performed.

When it is determined in step S1 to perform display that is easy to see for persons other than the surgeon, in step S2, the 3D image generation unit 24 arranges the angiographic fluoroscopic image on the display surface 12 of the display device **2.**

In step S3, the information processing apparatus 3 performs display surface display of the angiographic fluoroscopic image. Specifically, the 3D image generation unit 24 generates left-eye images and right-eye images for displaying the angiographic fluoroscopic image on the display surface 12, and the output control unit 25 displays the left-eye images and right-eye images on the display device **2.**

In step S4, the 3D image generation unit 24 highlights the angiographic fluoroscopic image (the region where display surface display is performed).

On the other hand, when it is determined in step S1 not to perform display that is easy to see for persons other than the surgeon, in step S5, the 3D image generation unit 24 performs virtual plate display of the angiographic fluoroscopic image. Specifically, the angiographic fluoroscopic image is projected onto a virtual plate arranged in virtual space so as to face directly toward the surgeon's viewing position, left-eye images and right-eye images for displaying this virtual plate on the display device 2 are generated, and the output control unit 25 displays the left-eye images and right-eye images on the display device **2.**

After display surface display is performed in step S3, or after virtual plate display is performed in step S5, in step S6, the 3D image generation unit 24 arranges the 3D model of blood vessels next to the angiographic projection image (virtual plate) in virtual space.

In step S7, the information processing apparatus 3 displays the 3D model in 3D on the display device **2.** Specifically, the 3D image generation unit 24 generates left-eye images and right-eye images such that the 3D model can be appropriately seen from the user's viewing position, and the output control unit 25 displays the left-eye images and right-eye images on the display device **2.**

As described above, in the information processing apparatus of the present technology, a display image (first display image) is generated based on the user's viewing position, the display image being an image in which 2D images such as angiographic fluoroscopic images are displayed in 2D on the display surface 12 and 3D models such as blood vessels are displayed in 3D, and being an image displayed on the display device **2.** By viewing the angiographic fluoroscopic images displayed on the display device 2 and further viewing the 3D models of blood vessels, the surgeon can intuitively understand the direction and angle of vascular pathways ahead of the current catheter position and perform precise treatment efficiently.

Since precise treatment is performed efficiently, the time required for surgery and treatment is shortened, and the burden on patients can be reduced. Also, effects such as efficient use of operating rooms and reduction of surgeon time and labor can be expected. When performing IVR treatment and the like, the patient's X-ray exposure time can be shortened, and the burden on the patient can be reduced.

Also, persons other than the surgeon can see the angiographic fluoroscopic images that the surgeon is viewing, and can assist the surgeon while viewing the angiographic fluoroscopic images.

### <3. Second Embodiment>

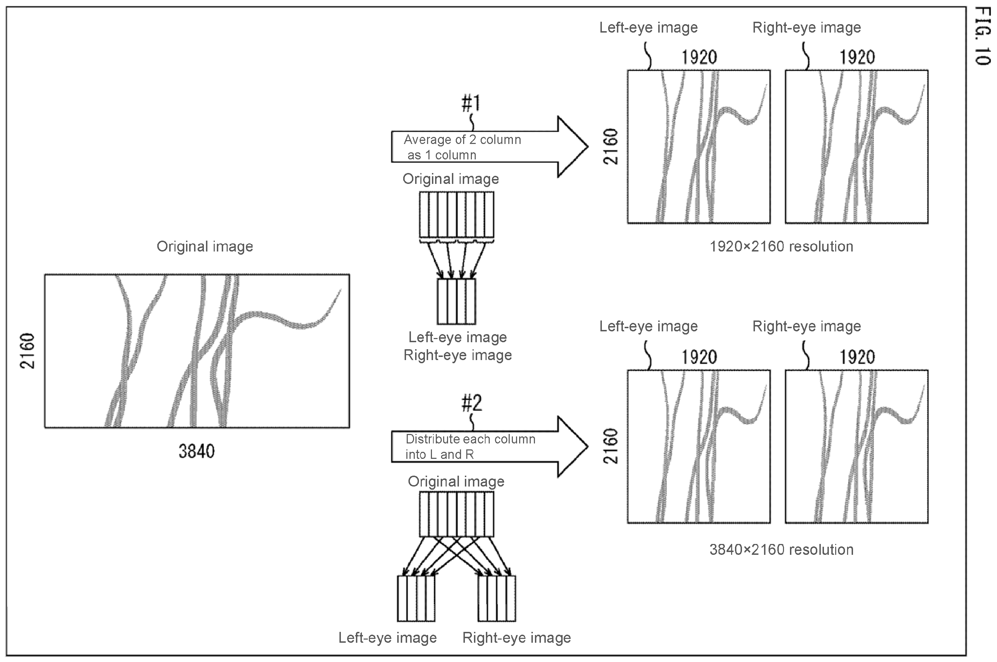

FIG. 10 is a diagram showing an example of resolution of images displayed on a spatial reproduction display.

When attempting to display a 2D image with a resolution of, for example, 3840×2160 on the display surface of a spatial reproduction display, normally, as shown by arrow #1 in FIG. 10, the original 2D image is down-converted by averaging pixel values every two pixels in the horizontal direction, and an image with a resolution of 1920×2160 is displayed as left-eye images and right-eye images. Therefore, the horizontal resolution of the displayed 2D image becomes half or less of the resolution of the original 2D image.

On the other hand, in the present technology, when a 2D image with a resolution of, for example, 3840×2160 is displayed on the display surface of a spatial reproduction display, as shown by arrow #2 in FIG. 10, left-eye images and right-eye images are generated by cutting out the original 2D image column by column and distributing them to left-eye images and right-eye images. By displaying the left-eye images and right-eye images with a resolution of 2160×1920 generated in this way, when viewed from the tracked surgeon's viewing position, a 2D image with a resolution of 3840×2160, which is the same as the resolution of the original 2D image, can be seen.

As described above, by generating left-eye images and right-eye images by cutting out the original 2D image column by column and distributing them to left-eye images and right-eye images, it becomes possible to increase the resolution of the displayed 2D image.

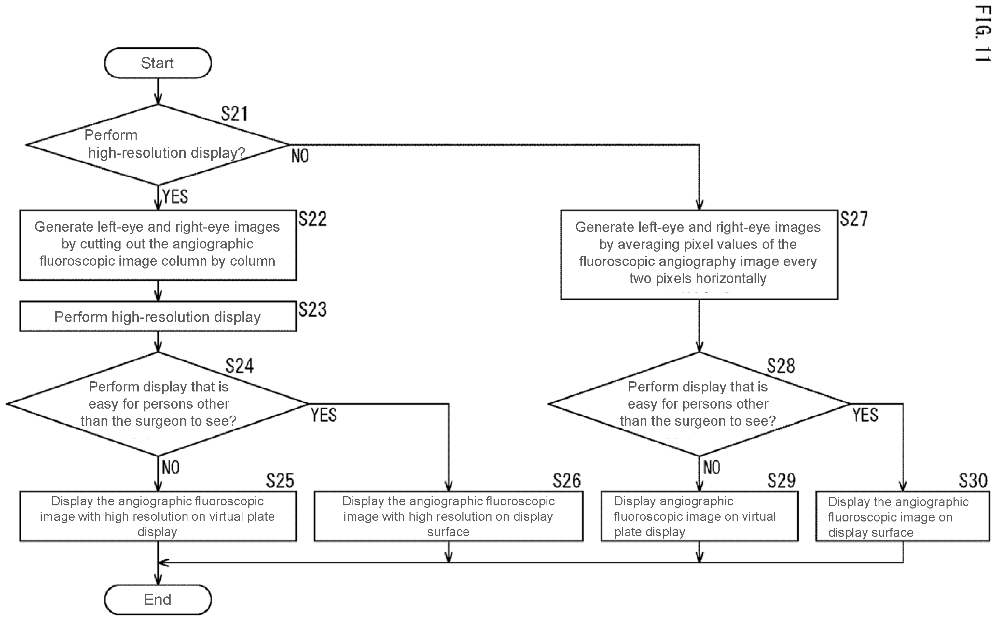

Next, with reference to the flowchart of FIG. 11, the processing performed by the information processing apparatus 3 when displaying high-resolution 2D images will be described. Here, the acquisition of angiographic fluoroscopic images and 3D models of blood vessels, the acquisition of operation content, and the detection of the user's viewing position are performed as appropriate. Note that FIG. 11 shows only the processing flow related to the display of angiographic fluoroscopic images for simplicity of explanation.

In step S21, the information processing apparatus 3 determines whether to perform high-resolution display of angiographic fluoroscopic images. Whether to perform high-resolution display of 2D images is determined, for example, based on operation content by the surgeon.

When it is determined in step S21 to perform high-resolution display of 2D images, in step S22, the 3D image generation unit 24 generates left-eye images and right-eye images by cutting out the angiographic fluoroscopic image column by column and distributing them to left-eye images and right-eye images.

In step S23, the information processing apparatus 3 performs high-resolution display of the angiographic fluoroscopic image. Specifically, the output control unit 25 displays the left-eye images and right-eye images generated in step S22 on the display device 2.

In step S24, the information processing apparatus 3 determines whether to perform display that is easy to see for persons other than the surgeon.

When it is determined in step S24 not to perform display that is easy to see for persons other than the surgeon, in step S25, the information processing apparatus 3 causes the display device 2 to perform virtual plate display of the angiographic fluoroscopic image at high resolution. Specifically, the 3D image generation unit 24 generates left-eye images and right-eye images for causing the display device 2 to perform virtual plate display of the angiographic fluoroscopic image at high resolution, and the output control unit 25 displays the left-eye images and right-eye images on the display device **2.**

On the other hand, when it is determined in step S24 to perform display that is easy to see for persons other than the surgeon, in step S26, the information processing apparatus 3 causes the display device 2 to perform display surface display of the angiographic fluoroscopic image at high resolution. Specifically, the 3D image generation unit 24 generates left-eye images and right-eye images for causing the display device 2 to perform display surface display of the angiographic fluoroscopic image at high resolution, and the output control unit 25 displays the left-eye images and right-eye images on the display device **2.**

When it is determined in step S21 not to perform high-resolution display, in step S27, the 3D image generation unit 24 generates left-eye images and right-eye images by averaging pixel values of the angiographic fluoroscopic image every two pixels in the horizontal direction.

In step S28, the information processing apparatus 3 determines whether to perform display that is easy to see for persons other than the surgeon.

When it is determined in step S28 not to perform display that is easy to see for persons other than the surgeon, in step S29, the information processing apparatus 3 causes the display device 2 to perform virtual plate display of the angiographic fluoroscopic image.

On the other hand, when it is determined in step S28 to perform display that is easy to see for persons other than the surgeon, in step S30, the information processing apparatus 3 causes the display device 2 to perform display surface display of the angiographic fluoroscopic image at high resolution.

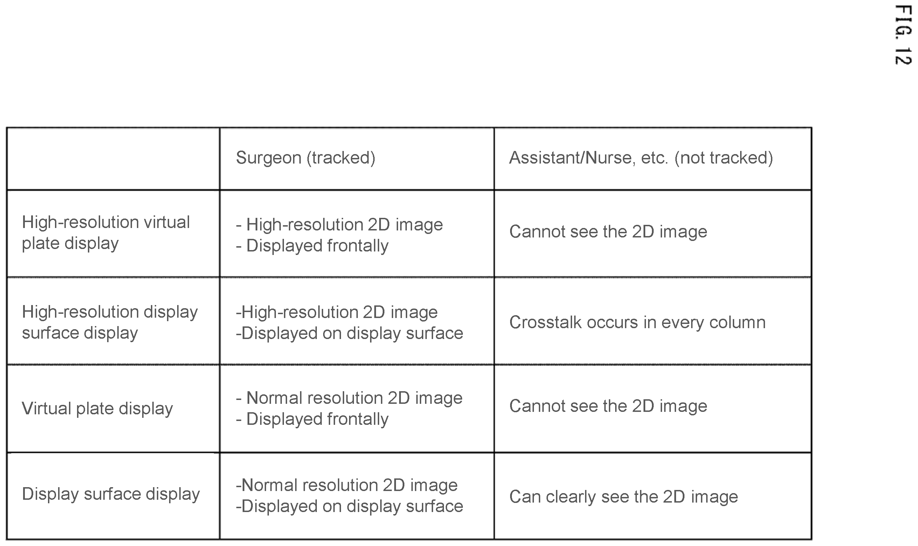

FIG. 12 is a diagram explaining 2D images that can be viewed by surgeons and persons other than surgeons in each display method.

As shown in FIG. 12, when high-resolution virtual plate display is performed, for the surgeon, a high-resolution 2D image appears to be arranged at a position directly facing their viewing position. In this case, when viewed from viewing positions other than the surgeon's, crosstalk occurs where left-eye images and right-eye images mix in one eye, so persons other than the surgeon, such as assistants and nurses, cannot see the 2D image.

When high-resolution display surface display is performed, for the surgeon, a high-resolution 2D image appears to be displayed on the display surface. In this case, persons other than the surgeon can see a 2D image with crosstalk occurring column by column.

When normal virtual plate display is performed, for the surgeon, a 2D image with normal resolution appears to be arranged at a position directly facing their viewing position. In this case, crosstalk occurs when viewed from viewing positions other than the surgeon's, so persons other than the surgeon cannot see the 2D image.

When normal display surface display is performed, for the surgeon, a 2D image with normal resolution appears to be displayed on the display surface. In this case, persons other than the surgeon can clearly see the 2D image.

### <4. Third Embodiment>

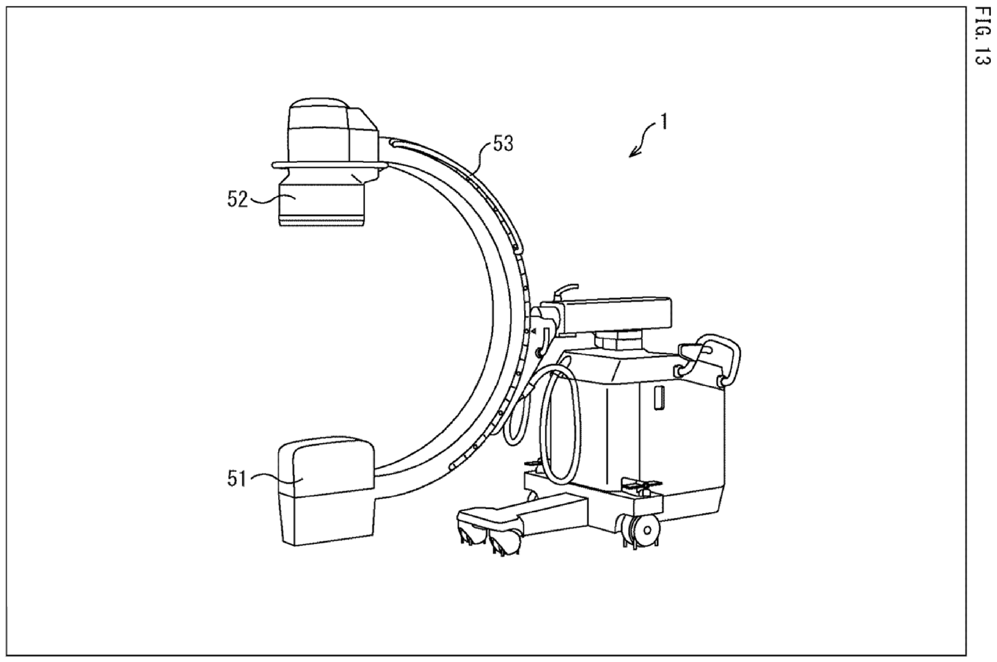

FIG. 13 is a diagram showing a configuration example of the C-arm 1.

As shown in FIG. 13, the C-arm 1 is configured, for example, by mounting an X-ray generation device 51 and a detection device 52 on an arm portion 53 so that they face each other. With the patient positioned between the X-ray generation device 51 and the detection device 52, **X-**rays are emitted from the X-ray generation device 51, and shadows of the patient's blood vessels and the like are projected onto the detection device 52, thereby capturing angiographic fluoroscopic images.

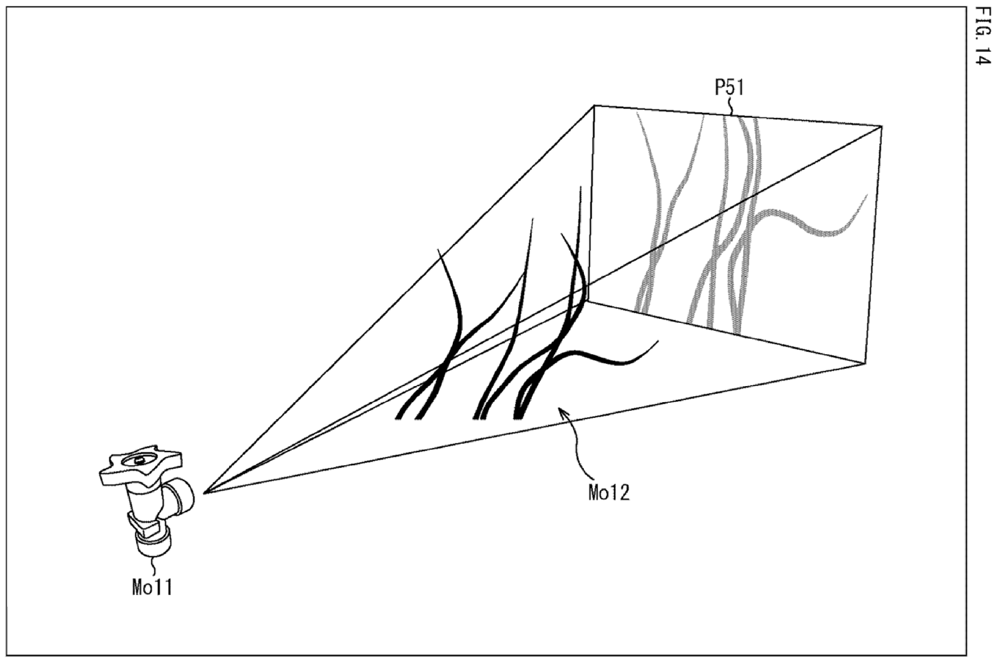

FIG. 14 is a diagram showing an arrangement example of 3D objects in virtual space.

In the example of FIG. 14, in virtual space, a 3D model Mo11 representing the X-ray generation device 51, a 3D model Mo12 of blood vessels, and an angiographic fluoroscopic image P51 (virtual plate) are arranged so as to reproduce the positional relationship between the X-ray generation device 51, the patient, and the detection device 52 shown in FIG. 13.

By displaying 3D images showing virtual space in which the 3D model Mo11 of the X-ray generation device, the 3D model Mo12 of blood vessels, and the angiographic fluoroscopic image P51 are arranged, the surgeon can intuitively refer to the portion of the 3D model Mo12 of blood vessels that corresponds to the portion of interest in the angiographic fluoroscopic image P51.

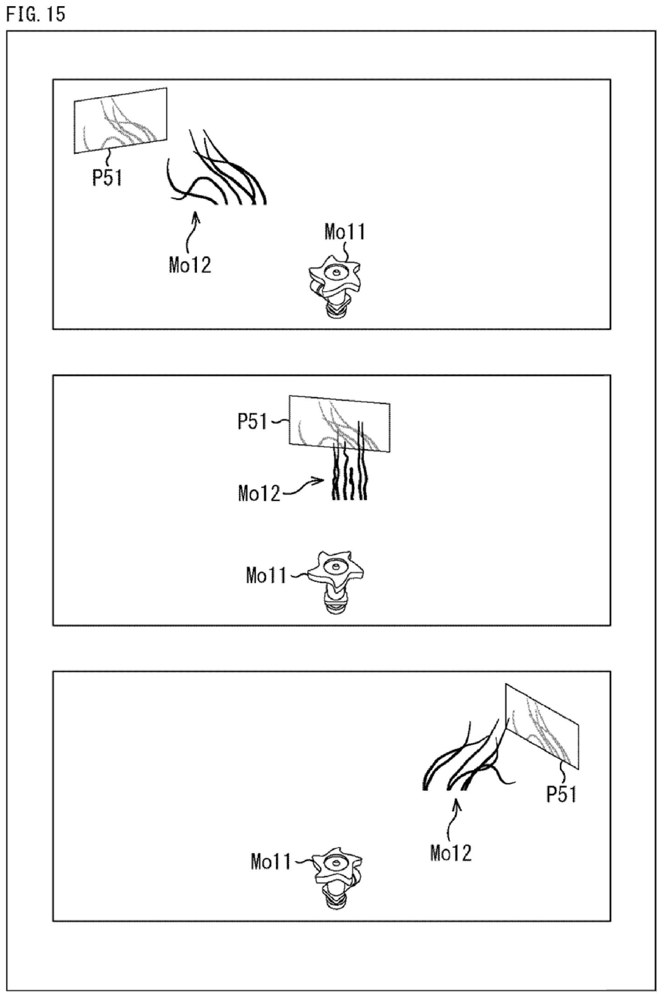

The surgeon can change the orientation of 3D objects arranged in virtual space by operating input devices such as a mouse and keyboard. For example, as shown in the order of the upper, middle, and lower sections of FIG. 15, the entire virtual space is displayed rotating around the 3D model Mo11 of the X-ray generation device according to the user's operation content.

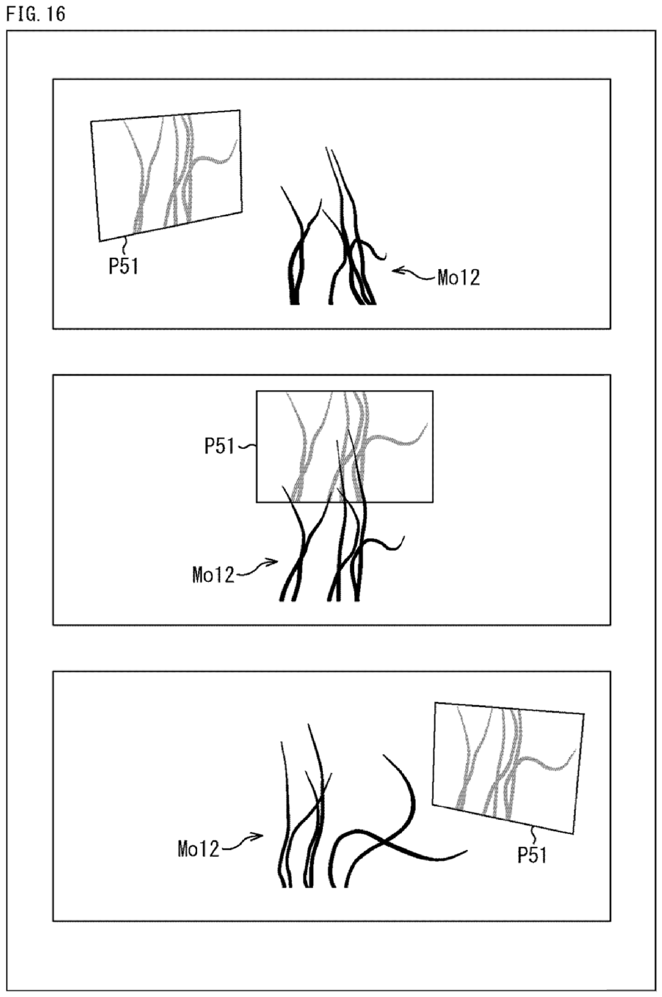

FIG. 16 is a diagram showing another example of arrangement of 3D objects in virtual space.

As shown in FIG. 16, only the 3D model Mo12 of blood vessels and the angiographic fluoroscopic image P51 may be arranged in virtual space. In virtual space, the 3D model Mo12 of blood vessels and the angiographic fluoroscopic image P51 are arranged so as to reproduce the positional relationship between the patient and the detection device 52.

In this case, as shown in the order of the upper, middle, and lower sections of FIG. 16, the entire virtual space is displayed rotating around the 3D model Mo12 of blood vessels according to the user's operation content.

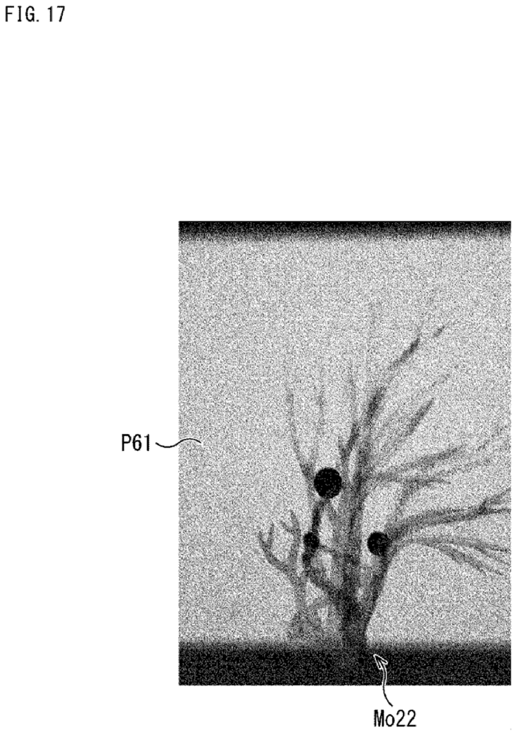

FIG. 17 is a diagram showing an actual display example of 3D objects.

As shown in FIG. 17, the 3D model Mo12 of blood vessels and the angiographic fluoroscopic image P61 arranged in virtual space in a positional relationship corresponding to the positional relationship between the patient and the detection device 52 are displayed on the display device **2.** Note that it is also possible to fix the arrangement of 3D objects and allow the surgeon to view the 3D model Mo22 of blood vessels and the like from various directions by changing their viewing position.

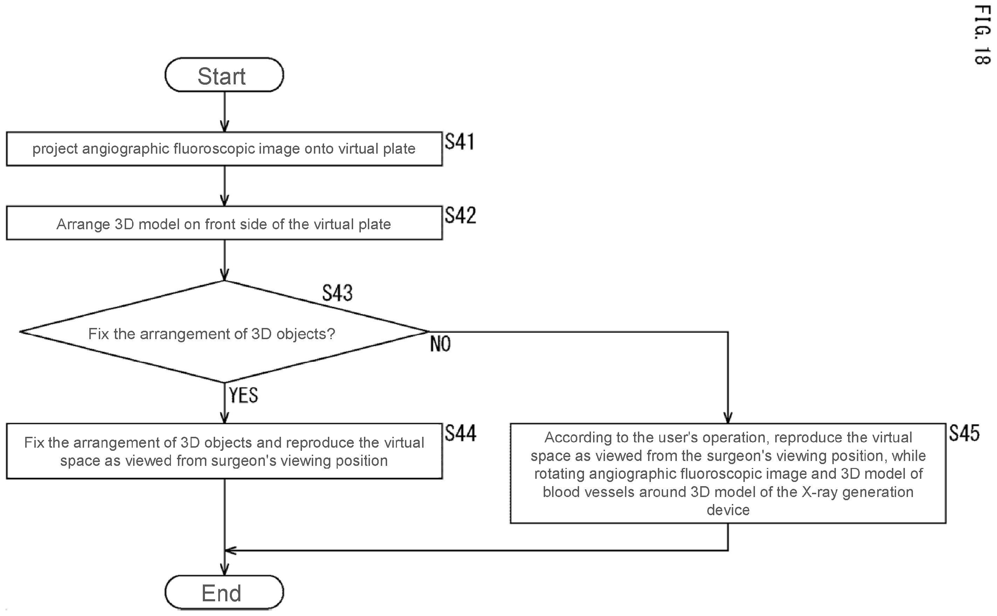

Next, with reference to the flowchart of FIG. 18, the processing performed by the information processing apparatus 3 when arranging 3D objects in a positional relationship corresponding to the positional relationship between the C-arm 1 and the patient will be described. Here, the acquisition of angiographic fluoroscopic images and 3D models of blood vessels, the acquisition of operation content, and the detection of the user's viewing position are performed as appropriate.

In step S41, the 3D image generation unit 24 projects the angiographic fluoroscopic image onto a virtual plate arranged in virtual space.

In step S42, the 3D image generation unit 24 arranges the 3D model of the X-ray generation device and the 3D model of blood vessels, for example, on the front side of the virtual plate. Here, the 3D model of the X-ray generation device, the 3D model of blood vessels, and the angiographic fluoroscopic image are arranged so as to reproduce the positional relationship between the X-ray generation device 51, the patient, and the detection device 52.

In step S43, the information processing apparatus 3 determines whether to fix the arrangement of 3D objects. Whether to fix the arrangement of 3D objects is set in advance by, for example, the surgeon.

When it is determined in step S43 to fix the arrangement of 3D objects, in step S44, the information processing apparatus 3 causes the display device 2 to reproduce virtual space as viewed from the surgeon's viewing position with the arrangement of 3D objects fixed. Specifically, the 3D image generation unit 24 generates left-eye images and right-eye images showing virtual space as viewed from the tracked surgeon's viewing position with the arrangement of 3D objects fixed, and the output control unit 25 displays the left-eye images and right-eye images on the display device **2.**

When it is determined in step S43 not to fix the arrangement of 3D objects, in step S45, the information processing apparatus 3 rotates the angiographic fluoroscopic image and the 3D model of blood vessels around the 3D model of the X-ray generation device according to the user's operation content. Also, the information processing apparatus 3 causes the display device 2 to reproduce virtual space as viewed from the surgeon's viewing position. Specifically, the 3D image generation unit 24 generates left-eye images and right-eye images showing virtual space as viewed from the tracked surgeon's viewing position with 3D objects or virtual space rotated according to the user's operation content, and the output control unit 25 displays the left-eye images and right-eye images on the display device **2.**

As described above, in the information processing apparatus of the present technology, a display image (second display image) is generated based on the user's viewing position, the display image being an image in which virtual space where virtual plates onto which 2D images such as angiographic fluoroscopic images are projected and 3D models such as blood vessels are arranged in a predetermined positional relationship is displayed in 3D, and being an image displayed on the display device **2.**

Note that each of the first embodiment, second embodiment, and third embodiment can naturally be implemented independently, or multiple embodiments may be combined.

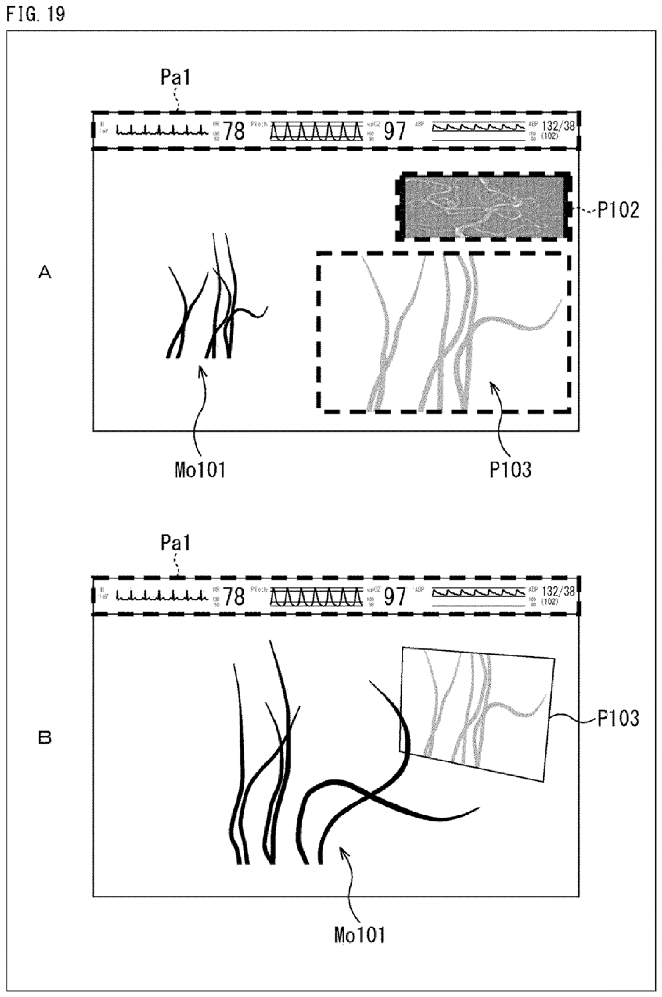

FIG. 19 is a diagram showing an example of a screen displayed on the display device **2.**

FIG. 19A shows a parallel display screen (first display image) in which a 3D model Mo101 of blood vessels and a reference image P102 and angiographic fluoroscopic image P103 of 2D images displayed on the display surface are arranged side by side. In the parallel display screen, for example, the 3D model Mo101 is displayed on the left side, the reference image P102 is displayed on the upper right side, and the angiographic fluoroscopic image P103 is displayed on the lower right side.

In the parallel display screen, as shown surrounded by dashed lines, the reference image P102 and the angiographic fluoroscopic image P103 (including the regions containing them) are highlighted.

Also, in the parallel display screen, a monitoring information part Pa1 is displayed in the upper portion, which presents time-related information such as electrocardiogram waveforms, heart rate, pulse waves, blood oxygen saturation, arterial pressure, and timers. The monitoring information part Pa1 is arranged on the display surface and highlighted, as shown surrounded by dashed lines.

FIG. 19B shows a superimposed display screen (second display image) in which the 3D model Mo101 of blood vessels and the angiographic fluoroscopic image P103 are arranged and displayed according to the positional relationship between the patient and the detection device 52. In the superimposed display screen as well, the monitoring information part Pa1 arranged on the display surface and highlighted is displayed in the upper portion.

For example, the output control unit 25 of the information processing apparatus 3 can mutually transition the screen displayed on the display device 2 between the parallel display screen and the superimposed display screen. For example, the screen displayed on the display device 2 seamlessly transitions from the parallel display screen to the superimposed display screen.

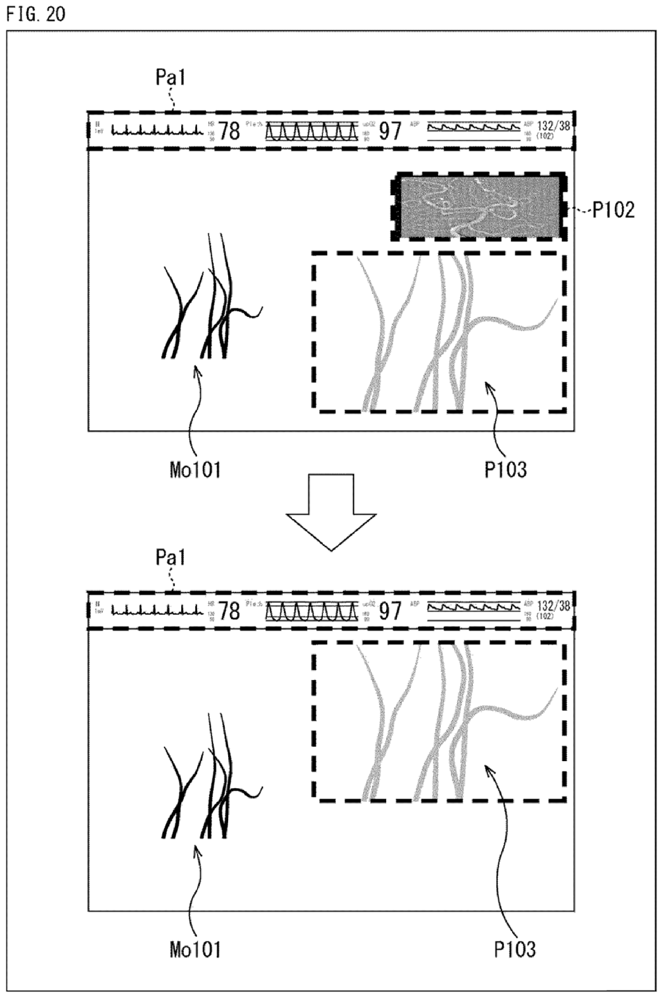

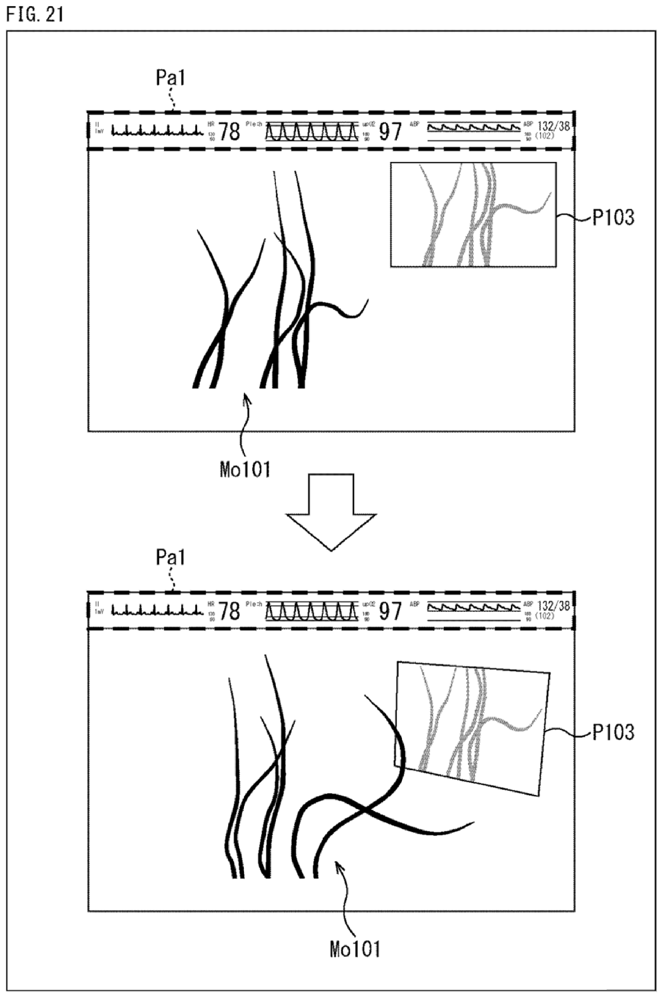

FIGS. 20 and 21 are diagrams explaining the flow of transition from the parallel display screen to the superimposed display screen.

When the parallel display screen shown in the upper part of FIG. 20 is being displayed and screen transition is instructed by, for example, the surgeon, first, as shown in the lower part of FIG. 20, the reference image P102 disappears and the angiographic fluoroscopic image P103 moves to the upper right side of the parallel display screen.

Next, as shown in the upper part of FIG. 21, the highlighting of the angiographic fluoroscopic image P103 ends, and the 3D model Mo101 and the angiographic fluoroscopic image P103 move within virtual space. Finally, as shown in the lower part of FIG. 21, the superimposed display screen is displayed.

As described above, by transitioning the screen displayed on the display device 2, the surgeon can, in certain situations, show angiographic fluoroscopic images to persons other than the surgeon and receive assistance from those persons, and in other situations, proceed with treatment while viewing 3D objects arranged in virtual space to understand the direction and angle of vascular pathways.

### <About Computers>

The series of processes described above can be executed by hardware or by software. When the series of processes is executed by software, the programs constituting the software are installed from a program recording medium into a computer incorporated in dedicated hardware or a general-purpose personal computer or the like.

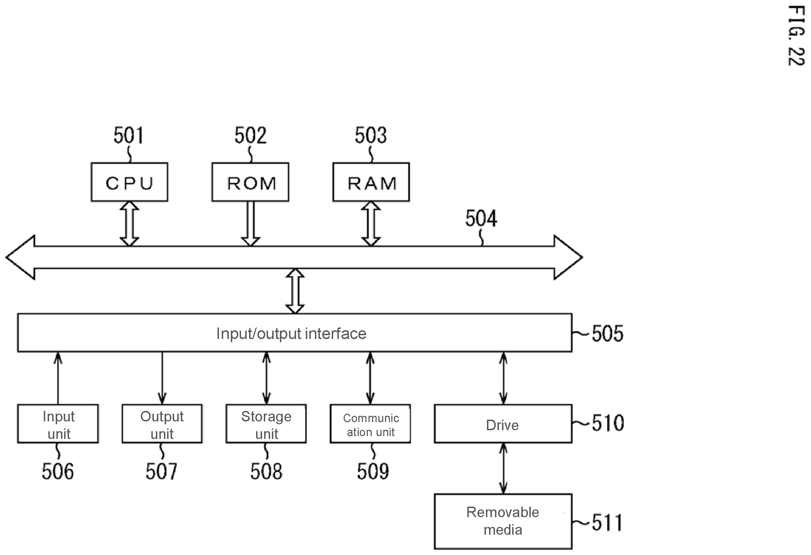

FIG. 22 is a block diagram showing a configuration example of computer hardware that executes the series of processes described above by programs.

A CPU (Central Processing Unit) 501, ROM (Read Only Memory) 502, and RAM (Random Access Memory) 503 are mutually connected by a bus 504.

An input/output interface 505 is further connected to the bus 504. Connected to the input/output interface 505 are an input unit 506 comprising a keyboard, mouse, and the like, and an output unit 507 comprising a display, speaker, and the like. Also connected to the input/output interface 505 are a storage unit 508 comprising a hard disk, non-volatile memory, and the like, a communication unit 509 comprising a network interface and the like, and a drive 510 that drives removable media 511.

In the computer configured as described above, the CPU 501 performs the series of processes described above by, for example, loading programs stored in the storage unit 508 into the RAM 503 via the input/output interface 505 and the bus 504 and executing them.

The programs executed by the CPU 501 are provided by being recorded on removable media 511, or via wired or wireless transmission media such as local area networks, the Internet, and digital broadcasting, and are installed in the storage unit 508.

Note that the programs executed by the computer may be programs in which processing is performed chronologically in the order described in this specification, or may be programs in which processing is performed in parallel or at necessary timing such as when calls are made.

Note that in this specification, a system means a collection of multiple components (devices, modules (parts), etc.), and it does not matter whether all components are in the same housing. Therefore, multiple devices housed in separate housings and connected via a network, and a single device having multiple modules housed in one housing are both systems.

The effects described in this specification are merely illustrative and not limiting, and there may be other effects.

The embodiments of the present technology are not limited to the above-described embodiments, and various modifications are possible within the scope that does not depart from the gist of the present technology.

For example, the present technology can take a cloud computing configuration in which one function is shared and jointly processed by multiple devices via a network.

Also, each step described in the above flowcharts can be executed by one device or can be shared and executed by multiple devices.

Furthermore, when one step includes multiple processes, the multiple processes included in that one step can be executed by one device or can be shared and executed by multiple devices.

### <Configuration Combination Examples>

The present technology can also take the following configurations.

(1) An information processing apparatus comprising:
   an image generation unit that generates a first display image based on a viewing position of a user of a spatial reproduction display, the first display image being an image in which a 2D image is displayed in 2D on a display surface and a 3D model is displayed in 3D, and being an image displayed on the spatial reproduction display having the display surface
(2) The information processing apparatus according to (1) ,
   in the first display image, a region including the 2D image is highlighted
(3) The information processing apparatus according to (1) or (2),
   the image generation unit generates the first display image by cutting out pixels of the 2D image column by column and distributing them to left-eye images and right-eye images that constitute the first display image
(4) The information processing apparatus according to (1) or (2) above. the image generation unit generates left-eye images and right-eye images that constitute the first display image by averaging pixel values of the 2D image every two pixels in the horizontal direction
(5) The information processing apparatus according to any one of (1) to (4),
   the image generation unit generates a second display image based on the user's viewing position, the second display image being an image in which virtual space where a virtual plate onto which the 2D image is projected and the 3D model are arranged in a predetermined positional relationship is displayed in 3D, and being an image displayed on the spatial reproduction display
(6) The information processing apparatus according to (5),
   further comprising a control unit that mutually transitions the image displayed on the spatial reproduction display between the first display image and the second display image
(7) The information processing apparatus according to (5) or (6),
   the 2D image includes an X-ray fluoroscopic image in which at least a part of a patient's living body is captured
(8) The information processing apparatus according to (7),
   the 3D model shows a shape of at least a part of the living body
(9) The information processing apparatus according to (8),
   in the virtual space, the virtual plate and the 3D model are arranged in a positional relationship corresponding to the positional relationship between a detection device that captures the X-ray fluoroscopic image and the patient
(10) An information processing method comprising:
   generating a first display image based on a viewing position of a user of a spatial reproduction display, the first display image being an image in which a 2D image is displayed in 2D on a display surface and a 3D model is displayed in 3D, and being an image displayed on the spatial reproduction display having the display surface.
(11) A program for causing a computer to execute processing, comprising:
   generating a first display image based on a viewing position of a user of a spatial reproduction display, the first display image being an image in which a 2D image is displayed in 2D on a display surface and a 3D model is displayed in 3D, and being an image displayed on the spatial reproduction display having the display surface.
(12) An information processing system comprising:
   an information processing apparatus having an image generation unit that generates a first display image based on a viewing position of a user, the first display image being an image in which a 2D image is displayed in 2D on a display surface and a 3D model is displayed in 3D, and
   a spatial reproduction display having the display surface and displaying the first display image.
(13) The information processing system according to (12),
   further comprising a sensor used for detecting the user's viewing position

### [Reference Signs List]

1 C-arm, 2 Display device, 3 Information processing apparatus, 11 Imaging device, 12 Display surface, 21 Capture unit, 22 UI processing unit, 23 Viewing position detection unit, 24 3D image generation unit, 25 Output control unit, 51 X-ray generation device, 52 Detection device, 53 Arm portion

## Claims

1. An information processing apparatus comprising:
an image generation unit that generates a first display image based on a viewing position of a user of a spatial reproduction display, the first display image being an image in which a 2D image is displayed in 2D on a display surface and a 3D model is displayed in 3D, and being an image displayed on the spatial reproduction display having the display surface.

2. The information processing apparatus according to claim **1,**
wherein in the first display image, a region including the 2D image is highlighted.

3. The information processing apparatus according to claim **1,**
wherein the image generation unit generates the first display image by cutting out pixels of the 2D image column by column and distributing them to left-eye images and right-eye images that constitute the first display image.

4. The information processing apparatus according to claim **1,**
wherein the image generation unit generates left-eye images and right-eye images that constitute the first display image by averaging pixel values of the 2D image every two pixels in the horizontal direction.

5. The information processing apparatus according to claim **1,**
wherein the image generation unit generates a second display image based on the user's viewing position, the second display image being an image in which virtual space where a virtual plate onto which the 2D image is projected and the 3D model are arranged in a predetermined positional relationship is displayed in 3D, and being an image displayed on the spatial reproduction display.

6. The information processing apparatus according to claim 5,
further comprising a control unit that mutually transitions the image displayed on the spatial reproduction display between the first display image and the second display image.

7. The information processing apparatus according to claim 5,
wherein the 2D image includes an X-ray fluoroscopic image in which at least a part of a patient's living body is captured.

8. The information processing apparatus according to claim 7,
wherein the 3D model shows a shape of at least a part of the living body.

9. The information processing apparatus according to claim 8,
wherein in the virtual space, the virtual plate and the 3D model are arranged in a positional relationship corresponding to the positional relationship between a detection device that captures the X-ray fluoroscopic image and the patient.

10. An information processing method, comprising:
generating a first display image based on a viewing position of a user of a spatial reproduction display, the first display image being an image in which a 2D image is displayed in 2D on a display surface and a 3D model is displayed in 3D, and being an image displayed on the spatial reproduction display having the display surface.

11. A program for causing a computer to execute processing, comprising:
generating a first display image based on a viewing position of a user of a spatial reproduction display, the first display image being an image in which a 2D image is displayed in 2D on a display surface and a 3D model is displayed in 3D, and being an image displayed on the spatial reproduction display having the display surface.

12. An information processing system comprising:
an information processing apparatus having an image generation unit that generates a first display image based on a viewing position of a user, the first display image being an image in which a 2D image is displayed in 2D on a display surface and a 3D model is displayed in 3D, and
a spatial reproduction display having the display surface and displaying the first display image.

13. The information processing system according to claim 12, further comprising a sensor used for detecting the user's viewing position.
